# EUROPEAN PATENT APPLICATION

(11) **EP 1 779 821 A1**
(43) Date of publication of application: **02.05.2007**
(21) Application number: 05110020.4
(22) Date of filing: 26.10.2005
(51) Int. Cl.: A61F 5/00

(54) **Adjustable gastric band**

(71) Applicant: Etervind AB, 192 69 Sollentuna (SE)
(72) Inventor: Axelsson, Robert, 563 91, Gränna (SE)
(74) Representative: Holme Patent A/S

(57) **Abstract**

An adjustable implantable band (1;31;32) for encircling an anatomical passageway to create an artificial stoma (6), comprises a flexible band (9) shapeable to a ring. The flexible band (9) is comprised of a flexible silicone tube (10) having a first lumen (28) enclosing at least one band or wire (11) of a material such as nitinol having a thermal shape memory. The flexible tube is a double lumen tube in which the nitinol band or wire (11) is enclosed in the first lumen and the second lumen is fluid inflateable from outside the patient to final adjustment of the internal diameter of the ring. The implantable band (1;31;32) are more easy to implant and more comfortable to the patient than known devices. Controlling and adjusting the internal diameter is very easy to perform, and just by adding cold saline or water to the lumens the ring will straighten out in an instant to be remove using any appropriate surgical keyhole procedure. The straightened-out ring will self-close upon injection of warm or hot fluid and adapt a ring-shape required for encircling a passageway.

## Description

This present invention relates to a surgically implantable band for encircling an anatomical passageway to create an artificial stoma. The band is of the kind comprising a flexible band shapeable to a ring and means for adjusting the internal diameter of the ring.

In particular the invention relates to a gastric band for encircling the stomach for the control of obesity.

Gastric bands have provided an effective alternative to gastric bypass, resections and other irreversible surgical weight loss treatments for the morbidly obese persons. The volume of the stomach is reduced in a surgical procedure in which a gastric band is arranged around an upper portion of the patient's stomach. A stoma is formed that is less than the normal interior diameter of the stomach. The stoma restricts food passing from the upper portion to a lower portion of the stomach and the digestive tract, so that food held in the upper portion of the stomach provides a feeling of fullness that discourages overeating. The patient will feel comfortably full with a small amount of food. And because of the slow emptying, the patient will continue to feel full for several hours reducing the urge to eat between meals.

Gastric bands are e.g. known from European patent applications EP1491167, EP1547549, and EP1303237, and from U.S. patent application US2005192531.

A feature common to all known gastric bands is the need of latching means. Respective mating latching components are provided at each end of the gastric band and used for providing the band with the shape required for forming a stoma suitable for the patient's need.

When latching the above known rings the surgeon must be very careful to avoid injuring the application site, e.g. the stomach wall. The tissue section of the stomach wall, between the mating latching components being drawn together by the surgeon for strapping the band, is at great risk being trapped and squeezed between said components. Blood supply is cut off, the tissue section degenerates and necrosis and infection are triggered. A further risk of damaging the tissue at the application site comes from the various surgical tools required for approaching the mating latching components into coupling relationship.

An inflatable balloon or cuff is often used for final adjustment of the diameter of the closed ring to the correct stoma size. However, the prior art gastric bands are not entirely circular in the closed stage, and as a result the balloon kinks during inflation. Areas of tissue are easily trapped at the kink and necrosis develops resulting in weakening of the stomach wall and even ulceration.

Adjustable gastric bands are intended implanted for very long periods of time including lifelong implantation in the human body. To operate the implant from outside most devices are provided with a subcutaneous fluid injection access port in communication with the adjustable gastric band. The shape and internal diameter of the band is adjusted using a needle for injecting a fluid through a silicone septum of the injection access port. Once the needle is removed, the septum seals against the hole by virtue of compressive load generated by the septum. Such injection access ports are known to the person skilled in the art and are not objects of the present invention.

In a first aspect according to the present invention is provided a surgically implantable band of the kind mentioned in the opening paragraph, said band being without sharp edges and being laparoscopically implantable and removable.

In a second aspect according to the present invention is provided a surgically implantable band of the kind mentioned in the opening paragraph having an adjustable internal diameter and is able to maintain a ring-shape without latching means.

In a third aspect according to the present invention is provided a surgically implantable band of the kind mentioned in the opening paragraph which provides an evenly distributed contact pressure to the stomach wall.

In a fourth aspect according to the present invention is provided a surgically implantable band of the kind mentioned in the opening paragraph which does not chafe, cut or clamp adjacent tissue.

The novel and unique feature whereby this is achieved according to the present invention is the fact that the flexible band shapeable to ring is comprised of a flexible tube having a first lumen enclosing at least one band or wire of a material having a shape memory.

The shape memory effect describes the process of restoring the original shape of a plastically deformed sample.

A material, which is both highly deformable and able to recover a pre-selected basic shape, e.g. a ring-shape, can be introduced through a very small incision in a laparoscopic procedure or other minimal invasive procedure. A flexible ring-shaped band or wire of memory shape material is easy to straight out. The straightened out tube-surrounded band or wire is applied adjacent a prearranged chosen zone of e.g. the stomach and allowed to recover its basic ring-shape in order to encircle the chosen zone and provide a stomach zone of reduced cross-section, the stoma. The zone is encircled in an instant. The upper portion of the stomach constitutes the new stomach reservoir of reduced size. After recovery of its basic ring-shape the memory shape material has the same diameter as before deformation and makes up the rigid backbone of the implantable band. The tube enclosing the memory shape material advantageously protects the stomach wall from being injured during shape recovery.

In a preferred embodiment the memory shape material is a thermal memory shape material. By changing the temperature of the memory shape material the three-dimensional shape can be affected and altered.

In juxtaposition to the first lumen, along the internal diameter of the ring, the flexible tube can be provided with an inflatable/deflatable second lumen for precision adjustment of the internal diameter of the ring-shaped implantable band. When the second lumen is inflated the internal diameter can be further reduced if desired. This situation arises if the weight loss has been insufficient or is smaller than expected. The second lumen exerts a radial gentle, equally distributed pressure on the stomach wall. All angular segments of the annulus are subjected to substantially the same radial force.

Another situation arises if the pressure on the annulus is to be relieved. If this situation arises the second lumen of the flexible tube can be deflated to increase the diameter of the ring. The negative pressure inside the second lumen advantageously reduces the pressure on the stomach wall.

The medium used for inflating the second lumen is typically a liquid such as saline or water. If hot saline is introduced into the first lumen the application of heat to a cooled band or wire will trigger said band or wire to recover is basic shape. The heat transfer is especially effective if the second lumen is the first lumen, since more fluid can be supplied to the flexible tube in less time. However, in this embodiment is can be problematic to control the radial pressure on the stomach wall.

A much better control of the radial pressure is obtained if the second lumen is put in fluid communication with the first lumen. Upon inflation, first the first lumen is inflated with warm fluid to allow the straightened out ring to recover its shape. Second, the internal diameter of the ring-shaped adjustable implantable band is adjusted, by filling the second lumen to the desired and required extent. However, since the two lumens communicate the process is very fast.

Access to the first and second lumens of the flexible tube can in a preferred embodiment be obtained via an inlet catheter and an outlet catheter having first ends coupled to an implantable fluid access port and second ends coupled to an inlet port and an outlet port, respectively, in the wall of the flexible tube. The fluid access port, which is subcutaneous implanted, is known to persons skilled in the art and will not be described further.

The inventors of the present invention have found that if a medical grade binary Nitinol alloy is selected as the material having a thermal shape memory excellent results are achieved.

Nitinol, which is an acronym for NIckel TItanium Naval Ordnance Laboratory, is a family of intermetallic shape memory materials exhibiting unique memory shape behaviour. Nitinol contains a mixture of app. 55% wt. nickel. The rest are titanium or titanium with an admixture of other elements, which are added to adjust or tune the material properties.

The exceptional properties of Nitinol are a result of a crystalline phase change known as "thermoelastic martensitic transformation". Below the transformation temperature, the alloy is soft and martensitic. Heating the material converts the material to its high strength, austenitic condition. The transformation from austenite to martensite (cooling) and the reverse cycle from martensite to austenite (heating) do not occur at the same temperature. There is a hysteresis curve for every Nitinol alloy that defines the complete transformation cycle. The shape memory effect is repeatable. ["Shape Memory Alloys", Edited by Hiroyasu Funakubo, Gordon and Breach Science Publishers, 1987, ISBN 2-88124-136-0].

The preferred material for the flexible tube is a medical grade silicone polymer. Such silicone polymers have been used for years and is recognized as biological acceptable. Silicone polymers have a very smooth surface and are not susceptible to ingrowth of tissue and leakage of encapsulated fluid.

The flexible band is manufactured so that at body temperature the basic shape is a substantially closed ring. The composition of the memory shape material is carefully chosen to comply with this condition.

In a first embodiment the adjustable implantable band can be designed so that a first end of the flexible band at least partly overlaps a second end of the band. As it may be both the memory shape material and the flexible tube overlaps or only the ends of the flexible tube overlaps. For example the ends can overlap at an interfacial angle.

In a second preferred embodiment the adjustable implantable band can be designed so that a first end and a second end of the flexible band of the band abut each other to form a complete enclosure.

In a third preferred embodiment the adjustable implantable band can be designed so that an interstice or small gap is left between a first end and a second end of the flexible band, thereby the leaving the option of further closing the implantable band at a later stage.

In all embodiments the ring is open. The adjustable implantable band is designed to resist a load of at least 5 kg at body temperatures between approximately 35 - 40°. If the stoma is in risk of being occluded by e.g. a lump of food, the 5 kg upper limit eventually will be exceeded and the flexible ring yields to allow passage of the lump.

The adjustable implantable band according to the present invention can be used as a gastric band or as an oesophageal band, for e.g. variceal ligation.

The invention also relates to a method for application of the band, including transforming and adjusting the three-dimensional shape of the adjustable implantable band according to the present invention.

The method comprises changing the crystalline phase of the implantable initially ring-shaped band including thermal shape memory material according to the present invention by cooling at or below a first temperature Tₘₐᵣ to provide a martensitic low temperature phase in which the band is straightened out, locating the martensitic straightened out ring at the application site, optionally during sustained cooling in a period, subjecting the martensitic ring to thermal application at or above a second temperature Tₐᵤₛ to provide an austenitic temperature phase in which the band recovers its ring-shape.

The austenitic temperature Tₐᵤₛ can be reached by simply flushing the first and/or the second lumen with a fluid having a temperature above Tₘₐᵣ. The first temperature Tₘₐᵣ of the implantable band in which the band is straightened out can be obtained by pre-cooling on ice or storing in a freezer.

Sustained cooling can be obtained by flushing the first and/or the second lumen with a fluid having a temperature below Tₘₐ.

Another way of reaching the austenitic temperature Tₐᵤₛ is upon contact with the application site of body temperature. Thermal conduction will promptly trigger recovery of the basic ring shape and the surgeon much take care that the implantable band not unintentionally is applied on the wrong application. It is therefore preferred in this embodiment to continuously supply cold fluid to the lumens.

Supply of fluid in at least the second lumen is made as described above using a subcutaneous implanted access port to adjust the internal diameter of the ring. Furthermore, via said access port the second lumen can be deflated or inflated to change the pressure in said second lumen, and accordingly adjust the radial pressure on the stomach wall, e.g. to avoid chafing.

The invention will be explained in greater detail below, describing only exemplary embodiments with reference to the drawing, in which
fig. 1 shows schematically a side view of an adjustable implantable gastric band according to the present invention installed around the stomach of a patient,
fig. 2 is a perspective view of a first embodiment of an adjustable implantable gastric band according to the present invention.
fig. 3a is a sectional view taken along the line III-III of fig. 2, illustrating the flexible band without pressure application of the second lumen,
fig. 3b shows the same applied with a positive pressure,
fig. 3c shows the same applied with a negative pressure,
fig. 4 corresponds to fig. 3a but the three adjacent memory shape metal rings are substituted with a flat memory shape band,
fig. 5 is a perspective view of the first embodiment shown in fig. 2, without the fluid access port, but cooled and straightened out ready for application in a patient,
fig. 6 shows schematically a cross-section of the flexible ring taken along the line VI-VI in fig. 2 of the inflated adjustable implanted band shown in fig. 3b,
fig. 7 is a perspective view of a second embodiment of an adjustable implantable gastric band according to the present invention, and
fig. 8 a perspective view of a first embodiment of an adjustable implantable gastric band according to the present invention.

Below, the invention is described by way of example on the assumption that the memory shape material is medical grade nitinol and the tube material is medical grade silicone.

However, the invention is not limited to these specific materials and other materials having similar or identical physical, mechanical and chemical characteristics may be used without departing from the scope of the present invention.

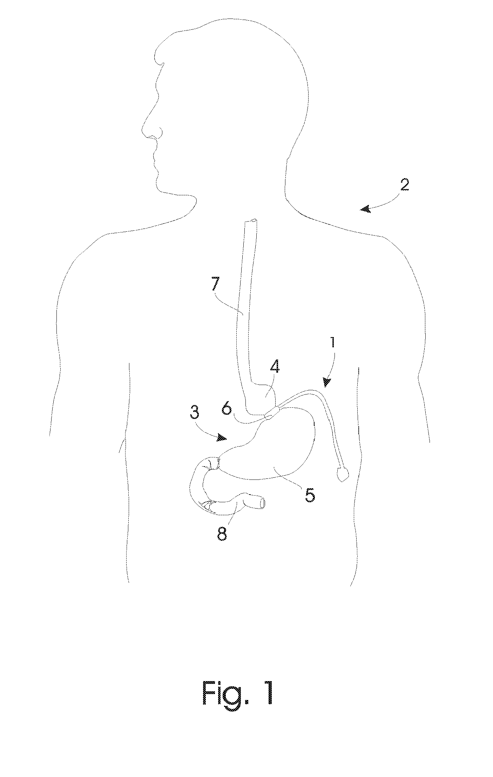

Fig. 1 teaches how the adjustable implanted band 1 according to the present invention is installed on the stomach 3 of a patient 2. The band 1 divides the stomach 3 in an upper portion 4 and a lower portion 5 intercommunicating via the stoma 6. Opposite the stoma 6 the upper portion 4 extend into the oesophagus 7, and the lower portion 5 extend into the small intestine 8.

Immediately after completion of the surgical implantation the upper portion 4, now being the new stomach, has a volumen of approximately 15 ml, said volumen expanding to approximately 60 - 80 ml.

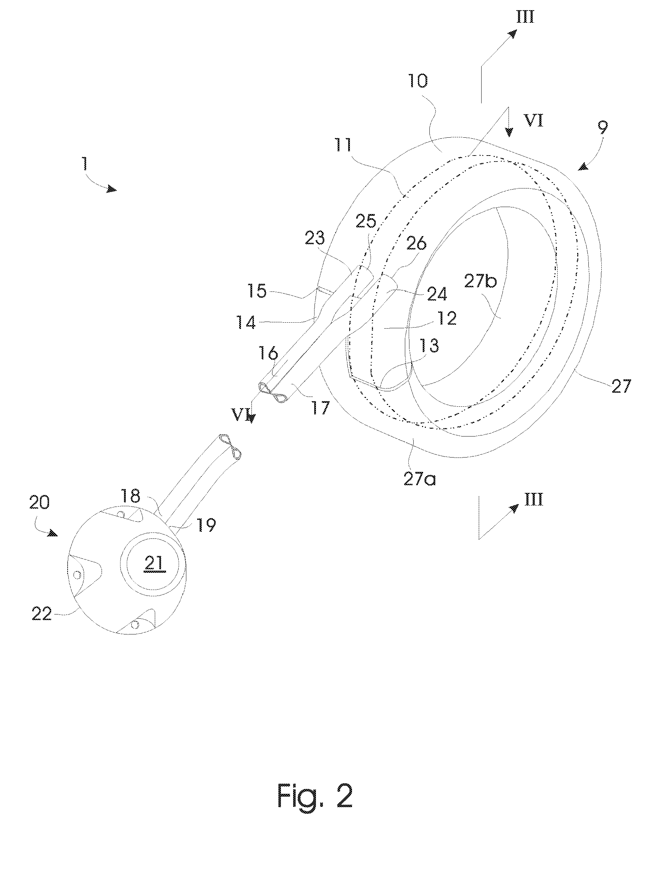

An enlarged perspective view of a first embodiment for a band 1 is shown in fig. 2.

The band 1 has a flexible band 9 consisting of a silicone tube or sleeve 10 surrounding a nitinol ring 11 (indicated in dotdash line). The flexible band 9 has a first end 12 with a protruding end part 13 and a second end 14 with a protruding end part 15. The protruding parts 13,15 have complement shapes enabling said protruding end parts 13,14 to both radially and circumferentially overlap each other to form a complete ring 9 or annulus for encircling the stoma 6. No latching means is provided nor required for holding the ring-shape.

An inlet catheter 16 and an outlet catheter 17 constitute fluid conduit means between a fluid access port 20 and the flexible band 9. The catheters 16,17 have first ends 18,19 connected to the fluid access port 20 and second ends 23,24 connected to the wall 27 of the silicone tube 10 via inlet port 25 and outlet port 26 to allow access to the lumens of said tube 10. A fluid such as saline is introduced via a self-sealing septum 21 of the fluid access port 20. Opposite the septum 21 the port 20 has a fixation face 22 provided for subcutaneous fixation by means of sutures at a distance from the stoma 6. The septum 21 is accessible and penetratable from outside the patient by means of a needle. The length of the catheters 16,17 is determined by the location of the fluid access port 20 and the application site of the band 1, taking in consideration that the implant must not cause any inconvenience to the patient at all.

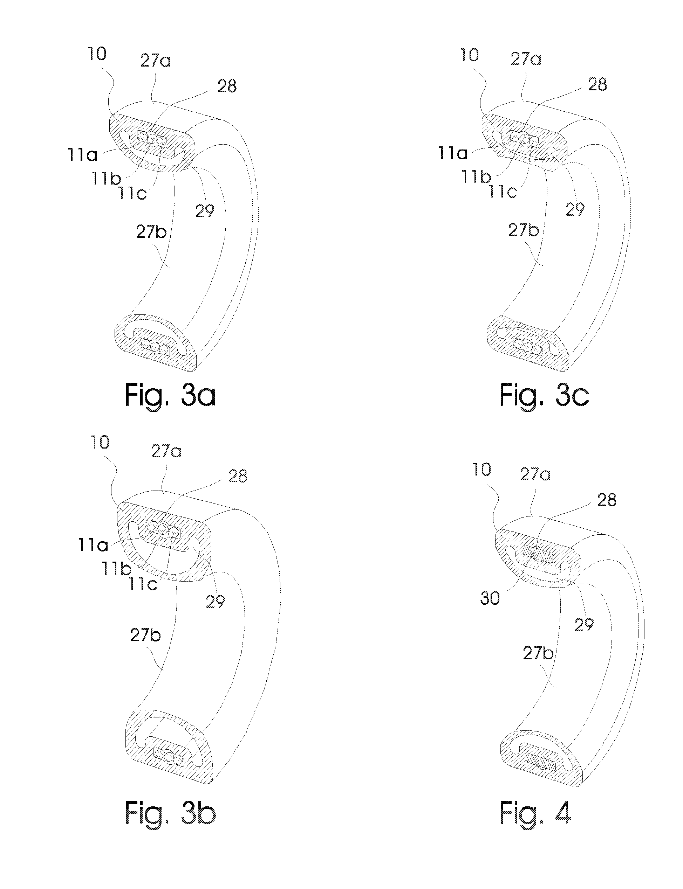

The internal design of the flexible band 9 is more clearly understood from figs. 3a, 3b, and 3c.

Fig. 3a shows a sectional view of the flexible band 9 composed of the silicone tube 10 and three nitinol rings 11a, 11b, 11c. The nitinol rings are enclosed in a flushable, first lumen 28 of the tube 10, said first lumen 28 extends along the exterior wall 27a in the ring condition of the flexible band 9. A flushable second lumen 29 is defined concentric with and in fluid communication with the first lumen 28a along the interior wall 27b in the ring condition of the flexible band 9. In fig. 3a the second lumen 29 is empty and no pressure is applied to the interior wall 27b of the tube 10.

In fig. 3b the second lumen 29 is inflated as a balloon with e.g. saline to reduce the internal diameter of the flexible ring 9. Due to the elasticity of the wall 27 of the silicone tube 10, especially the interior wall 27b, the balloon expands to a certain degree over the entire internal circumference without kinking. For the same reason, and because the band 1 is substantially closed the inflating pressure is distributed evenly along the entire annulus. No tissue is trapped and the expanded band 1 does not chafe the stoma 6.

Should relaxation of the applied pressure be needed, the condition of fig. 3b is reached by deflating the second lumen 29. A negative pressure is used to alleviate stress and load, and prevent erosion of the stomach tissue around the stoma 6.

Within the scope of the present invention the memory shape ring 11 can be made with a plurality of cross-section and be composed of one or more elements.

Fig. 4 corresponds substantially to fig. 3c. The only difference is that the three nitinol rings 11a, 11b, 11c are substituted by a flat nitinol band 30.

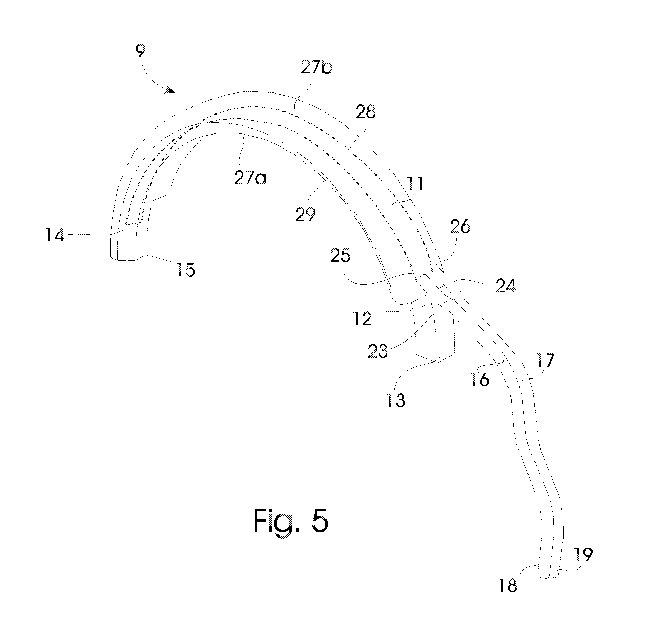

In fig. 5 the temperature T of the adjustable implantable band 1 has been reduced to below martensitic temperature Tₘₐᵣ, e.g. a Tₘₐᵣ of 4° - 6°C, to provide the flexible ring-shaped band 9 shown in fig. 2 with an elongated three-dimensional martensitic phase shape 9', which allows the band 1 to be implanted through a very small abdominal incision. As long as the temperature T is kept below Tₘₐᵣ, the elongated three-dimensional arc shape is maintained.

As an example a 2 mm thick nitinol ring [55, 66w% nickel, ≤ 0,05w% carbon, ≤ 0,05w% of the components cobalt, cobber, iron, molybdenum, niobium, silicon and wolfram, the rest is titanium], which has a basic medium diameter of app. 42 mm. The ring opens at 5°C into an elongated arc, in which the radius of a circle including said arc has a diameter of app. 100 mm.

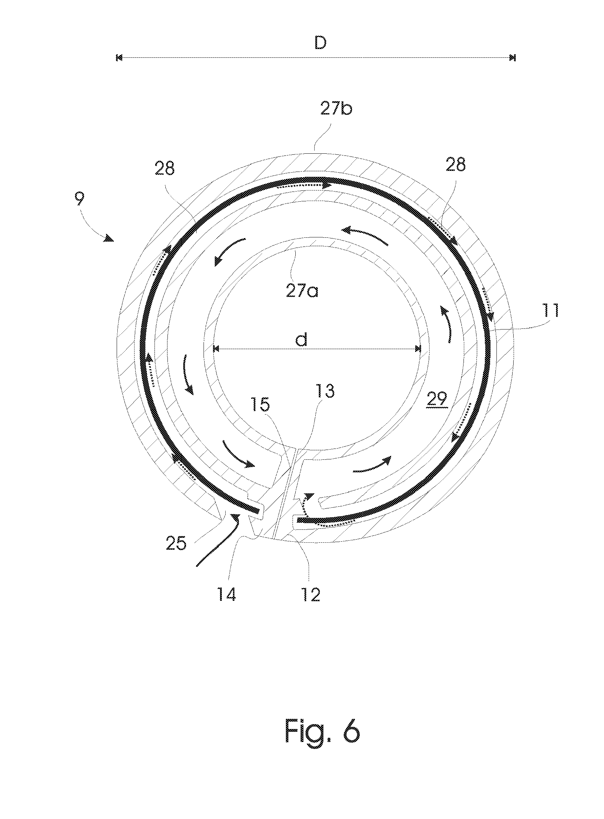

To illustrate the fluid communication between the first lumen 28 and the second lumen 29 references are now made to fig. 6 showing a cross-section of an inflated fluid-filled, flexible ring taken along the line VI-VI in fig. 2. including the inlet port 25.

Saline at a temperature of app. 40°C has been injected through the inlet port 25, as indicated by the arrow A. The saline flows through the first lumen 28 as shown with dot-arrows to heat the nitinol ring 11 to trigger self-closing of the flexible martensitic band 9' to provide the ring-shape as shown. The first lumen 28 communicates with the second lumen 29 and the injected saline continues into the second lumen 29, which is expanded until the desired final internal diameter d is reached. The thickness of the internal wall 27b of the silicone tube 10 can be designed with various degrees of material thickness so further ensure that kinking is avoided.

The inflated implantable adjustable band 1 has an external diameter D when implanted.

It is also possible to extract saline after implantation, preferably until a negative pressure is created inside at least the second lumen 29. In this condition the load on the internal stoma wall 27b is reduced. As a result, the risk of pathological conditions such as chafing, irritation, inflammation, erosion, necrosis and ulceration are most unlikely to happen. Moreover, the risk of the patient feels the band 1 is considerable reduced.

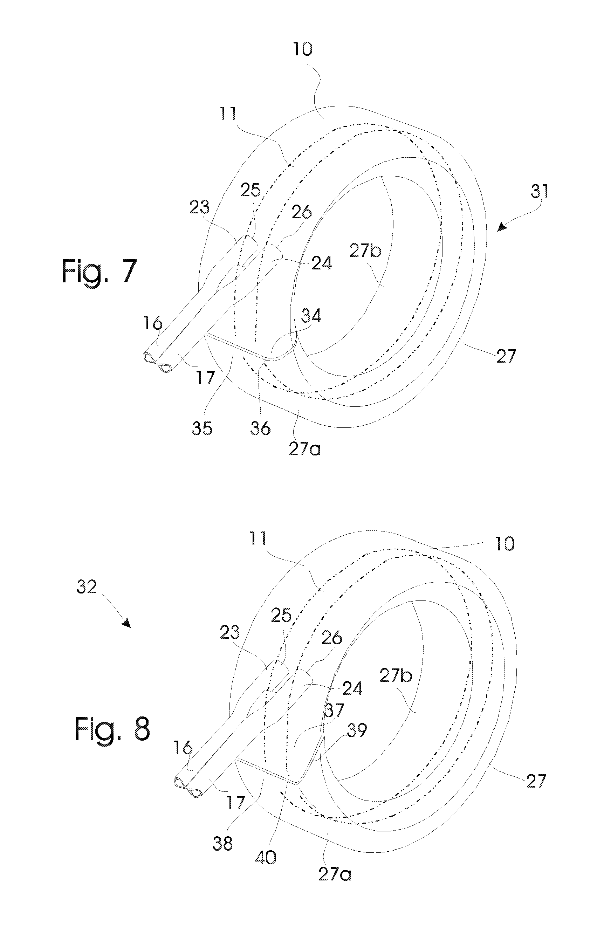

Fig. 7 shows a very simple second embodiment 31 of the adjustable implantable band according to the present invention. The band 31 corresponds substantially to the first embodiment 1 and for like parts are used same numerals.

In the second embodiment 31 the first end part 34 either abuts the second end part 35, or leaves a small gap 36 open between the end parts 34,35. The second embodiment is very simple to manufacture and is often preferred if the memory shape material is the continuous band 30 shown in fig. 4.

Fig. 8 shows a third preferred embodiment 32, which corresponds substantially to the first embodiment 1 and for like parts are used same numerals.

In the third embodiment 32 the end parts 37,38 overlap at complement slanting end faces 39,40 to define a perfect ring, which is able to flex against pressure and still be closed.

Common to the embodiments shown the flexible tube is a double lumen tube in which the nitinol band or wire (11) is enclosed in the first lumen 28, and the second lumen 29 is inflateable from outside the patient to final adjustment of the internal diameter d and to a certain extent also the external diameter of the flexible ring 9. Within the scope of the present invention the first and second lumens can be one and the same lumen enclosing the band or wire 10.

Modifications and other uses of the above adjustable implantable bands according to the present invention are within the scope of the appended set of claims.

During load and stress tests the bands has been subjected to over 200.000 loads contributions of 5 kg. No permanent deformation, metal fatigue, brittleness or leakage from then lumens of the tube has been observed.

The adjustable implantable band according to the present invention is seen by the present inventor to be superior to known bands. They are more easy to implant and more comfortable to the patient. Controlling and adjusting the internal diameter is very easy to perform, and just by adding cold saline or water to the lumens the ring will straighten out in an instant to be remove using any appropriate surgical keyhole procedure.

## Claims

1. An adjustable implantable band (1;31;32) for encircling an anatomical passageway ((5) to create an artificial stoma (6), comprising
- a flexible band (9) shapeable to a ring,
- means for adjusting the internal diameter (d) of the ring,
**characterised in that**
- the flexible band (9) shapeable to a ring is comprised of a flexible tube (10) having a first lumen (28) enclosing at least one band or wire (11) of a material having a shape memory.

2. An adjustable implantable band (1;31;32), according to claim 1, **characterised in that** the shape memory material is a thermal shape memory material.

3. An adjustable implantable band (1;31;32) according to claim 1 or 2, **characterised in that** in juxtaposition to the first lumen (28), along the internal diameter (d) of the ring, the flexible tube (10) has an inflatable/deflatable second lumen (29) for precision adjustment of the internal diameter (d) of the ring-shaped implantable band (1).

4. An adjustable implantable band (1;31;32) according to claim 2, **characterised in that** the second lumen (29) is the first lumen (28) or is in fluid communication with the first lumen (28).

5. An adjustable implantable band (1;31;32) according to claim 2 or 3 **characterised in that** the adjustable implantable band (1) further comprises an inlet catheter (16) and an outlet catheter (17) having first ends (18,19) coupled to an implantable fluid access port (20) and second ends (23,24) coupled to an inlet port (25) and an outlet port (26), respectively, in the wall (27) of the flexible tube (10).

6. An adjustable implantable band (1;31;32) according to any of the preceding claims 1 - 4, **characterised in that** the material having a thermal shape memory is a medical grade binary Nitinol alloy.

7. An adjustable implantable band (1;31;32) according to any of the preceding claims 1 - 5, **characterised in that** the flexible tube (10) is made of a medical grade silicone polymer.

8. An adjustable implantable band (1;31;32) according to any of the preceding claims 1 - 6, **characterised in that** at body temperature the flexible band (9) is shaped as a substantially closed ring, wherein
- a first (12;37) end of the flexible band (10) at least partly overlaps a second end (14;38) of the band,
- a first end (34) and a second end (35) of the flexible band (9) abut each other, or
- an interstice or gap is left between a first end (34) and a second end (35) of the flexible band (9).

9. An adjustable implantable band (1;31;32) according to any of the preceding claims 1 - 7, **characterised in that** the band (1) is a gastric band or an oesophageal band.

10. A method for adjusting the three-dimensional shape of the adjustable implantable band (1;31;32) according to any of the claims 1 - 8, **characterised in that**, the method comprises
- changing the crystalline phase of the initially ring-shaped band or wire (11) of thermal shape memory material by cooling at or below a first temperature Tₘₐᵣ to provide a martensitic low temperature phase,
- locating the martensitic ring (9') at the application site, optionally during sustained cooling in a period,
- subjecting the martensitic ring (9') to thermal application at or above a second temperature Tₐᵤₛ to provide an austenitic temperature phase in which the band (9) recovers its ring-shape.

11. A method according to claim 9, **characterised in that** the austenitic temperature Tₐᵤₛ is reached by flushing the first (28) and/or the second lumen (29) with a fluid having a temperature above Tₘₐᵣ.

12. A method according to claim 9, **characterised in that** the sustained cooling is obtained by flushing the first (28) and/or the second lumen (29) with a fluid having a temperature below Tₘₐᵣ.

13. A method according to any of the claims 9, 10 or 11 **characterised in that** the austenitic temperature Tₐᵤₛ is reached when contacting the application site having body temperature.

14. A method according to claim 9, **characterised in that** at least the second lumen (29) is inflated or deflated via a subcutaneous access port (20) to adjust the internal diameter of the ring (9).

15. A method according to claim 9, **characterised in that** the second lumen (29) is deflated to create a negative pressure in said second lumen (29).
